# EUROPEAN PATENT SPECIFICATION

(11) **EP 0 437 816 B1**
(45) Date of publication and mention of the grant of the patent: **26.07.1995**
(21) Application number: 90125248.6
(22) Date of filing: 21.12.1990
(51) Int. Cl.: D21H 21/22, D21H 23/04, A61L 15/00

(54) **Superabsorbent wet-lay nonwoven product**
Superabsorbierende, nassgelegte, nichtgewebte Faserbahn
Produit non tissé super-absorbant fabriqué par voie humide

(30) Priority: 16.01.1990 US 464798
(43) Date of publication of application: 24.07.1991
(73) Proprietor: HOECHST CELANESE CORPORATION, Somerville, N.J. 08876 (US)
(72) Inventor: Kim, Dai W., Chatham, New Jersey (US); Nielsen, Steven F., Charlotte, North Carolina (US)
(74) Representative: von Kreisler, Alek, Dipl.-Chem.

(56) References cited:
- EP-A- 0 257 951
- EP-A- 0 273 075
- DATABASE WPIL, no. 90-357249,Derwent Publications Ltd, London, GB,& JP-A-02227435 (SANYO CHEM.LIM.LTD. & HOECHST CELANESE CORP.)

## Description

### Background of Invention

### 1. Field of Invention

This invention discloses water absorbent polymer articles and processes for their production. More particularly, this invention discloses a superabsorbent nonwoven product prepared by a wet-lay process.

### 2. Prior Art

Disposable articles, such as diapers, sanitary napkins, incontinence garments, bandages and the like, employ pads, batts or other absorbent material to absorb and retain liquids. Significant effort has been devoted to increasing the amount of liquids that can be retained by these absorbent articles. By increasing the absorbent capacity of these articles, the physical dimensions, such as thickness, of the articles can be reduced.

For example, various absorbent products have incorporated within their structure certain water absorbent materials to increase the liquid retention capacity of these products. In certain configurations, these water absorbent materials tend to migrate to particular areas of the products. For example, U.S. Patent No. 4,699,823 describes a non-layered absorbent insert having a Z-directional water absorbent concentration gradient which increases the absorbent capacity of the material.

U.S. Patent No. 3,888,257 describes a disposable absorbent article which includes a matrix of fiberized wood pulp. Hydrocolloid polymer particles are located in a three-dimensional dispersion in a central zone or strip of the fiberized matrix.

U.S. Patent No. 4,333,462 describes an absorbent structure which includes an absorbent batt containing two reservoirs. The first reservoir opens toward the cover of the article. The second reservoir, located below the first reservoir, contains particles of water absorbent material.

U.S. Patent No. 4,381,783 describes an absorbent article which includes an absorbent layer having at least one discrete internal pocket. The pocket contains a uniform admixture of discrete water absorbent particles and discrete introfying particles.

U.S. Patent No. 4,381,782 discloses highly absorbent, low density fibrous structures such as webs or batts which include mixtures of powdered or microcrystalline hydrogel preparations with surfactant-treated filler material. The hydrogel preparations are located in a transverse band or strip extending across the width of the absorbent batt.

U.S. Patent No. 4,333,456 discloses a sanitary towel which includes an absorptive hydrophilic fiber filling material. An internal core of the filling material has an insert which contains highly absorptive polymers.

British Patent No. 1,406,615 describes absorbent pads which include absorptive gelling agents, such as cellulose ethers, cellulose esters and acetyl starch. The gelling agent is impregnated into selected central areas of the pad.

In addition, the following patents disclose absorbent pads which contain centralized layers or zones composed of materials which are more absorbent than the other portions of the pad: U.S. Patent Nos. 3,073,309 and 3,121,427.

U.S. Patent No. 3,670,731 discloses an absorbent dressing which includes a water soluble hydrocolloid composition. The hydrocolloid can be intermixed with the fibers of an absorbent pad or may be located in a discrete layer along a major surface of the absorbent article.

Absorbent pads have also included water absorbent particles disposed in strip-type patterns. For example, U.S. Patent No. 3,968,798 describes an absorbent pad in a C-fold configuration which has a middle portion thereof loaded with a uniform dispersion of hydrocolloid polymer particles.

U.S. Patent No. 4,410,324 describes a disposable diaper which has an absorbent pad folded intermediate the ends to provide increased absorbent material in the crotch region. Hydrocolloid material can also be located in strips along marginal portions of the absorbent pad.

In addition, absorbent materials have been enclosed in foams and other types of materials. For example Japanese Patent Application Kokai, No. 57-92,032 (1982)discloses a polyurethane foam that contains a particularly useful water absorbent polymer wherein the percentage of the air bubble formation is in the range of 1 to 60 percent, the diameter of the cells is in the range of 200 to 400 »m and the size of the water absorbent resin is in the range from about 200 to 400 »m.

In addition, a particularly useful biodegradable, high water absorbent polymer has been disclosed in U.S. Patent No. 4,076,663. While the resins of this patent do show increased water absorbency, their use has been limited to mixing them with sanitary napkins, diapers and other such products.

None of these patents disclose the combination of a water absorbent resin with conventional fibers in a slurry to produce a water absorbent nonwoven material.

U.S. Patent Nos. 4,454,268, 4,337,181 and 4,133,784 disclose various types of films partially comprised of water absorbent polymers. While these patents disclose starch-based, water absorbent polymers prepared from a combination of starch and ethylene acrylic acid copolymers, they fail to disclose the particular type of water absorbent polymer disclosed herein or the mixture of a water absorbent polymer with conventional fibers to form a water absorbent nonwoven material.

Also, U.S. Patent No. 3,669,103 discloses water swellable, water insoluble polymeric sorbents for the absorption of aqueous fluids wherein said polymeric sorbents are lightly crosslinked polymers. This patent discloses the use of a water insoluble polyurethane foam as a support for a polymeric absorbent. However, it fails to disclose the use of a water absorbent polymer in general, the water absorbent polymer disclosed herewith, particularly, or the use of a water absorbent material with conventional fibers to form a nonwoven water absorbent material.

U.S. Patent No. 4,792,326 discloses rapidly disintegrating paper tubes used in the preparation of tampon tubes. One of the superabsorbent materials disclosed in this patent could be used with this invention. However, this patent fails to disclose the use of the material in the formation of a nonwoven superabsorbent material.

U.S. Patent No. 4,604,313 discloses the selective layering of a water absorbent material in meltblown substrates. While this patent discloses the use of a water absorbent material in combination with certain fiberous material, it does not disclose the combination of the water absorbent material with conventional fibers by a wet-lay process for the preparation of a nonwoven water absorbent material.

Accordingly, it is an object of this invention, to prepare a superabsorbent nonwoven material.

It is a further object of this invention to prepare a superabsorbent nonwoven material useful in disposable articles such as diapers, sanitary napkins, incontinence garments, and the like.

It is a further object of this invention to prepare a superabsorbent nonwoven material that is easy and inexpensive to produce.

It is a still further object of this invention to prepare a superabsorbent nonwoven material by a wet-lay process.

It is a still further object of this invention to prepare composite materials by a wet-lay process combining a superabsorbent product with fiberous materials.

These and other objects as well as the scope, nature and utilization of this invention will become apparent to those skilled in the art from the following Detailed Description and appended Claims.

### Summary of the Invention

A superabsorbent nonwoven wet-lay material obtainable by the process of:
a) blending superabsorbent polymer particulates with a liquid to form a superabsorbent polymer particulate slurry wherein the concentration of the superabsorbent material is from 0.001 to 40.0 wt% of the superabsorbent/liquid mixture, and wherein said superabsorbent polymer is a polymer of water soluble acrylic or vinyl monomers which are slightly crosslinked with a polyfunctional reactant;
b) mixing fibers with the superabsorbent polymer particulate slurry to form a superabsorbent polymer slurry/fiber mixture;
c) filtering the superabsorbent polymer slurry/fiber mixture to remove a portion of the liquid; and
d) drying the superabsorbent polymer fiber material at a temperature not in excess of 250°C to form a nonwoven wet-lay superabsorbent material.

The absorbent article in this invention can absorb water up to 350 times the weight of the superabsorbent material contained within the article. It can be highly useful in those areas where high fluid absorbence is important such as for diapers, sanitary napkins, box linings, food packaging and general packaging material. Since this material is nonwoven, it can also be broken up and combined with less absorbent materials for use in diapers, sanitary napkins, incontinence devices, wound dressing and other similar products.

### Detailed Description of Invention

The superabsorbent nonwoven material is prepared by the process of blending superabsorbent particulates with a liquid to form a slurry, mixing fibers with the slurry to form a superabsorbent particulate slurry/fiber mixture, filtering the slurry mixture and drying the superabsorbent particulate slurry/fiber mixture to form a nonwoven superabsorbent material.

The superabsorbent polymer particulates useful in this invention are water insoluble but water swellable materials which are capable of absorbing many times their own weight of water or aqueous solutions. These superabsorbent polymers are polymers of water soluble acrylic or vinyl monomers which are slightly crosslinked with a polyfunctional reactant. Such superabsorbent polymers include polyvinylpyrrolidone, sulfonated polystyrene, polysulfoethyl acrylate, poly(2-hydroxyethylacrylate) polyacrylamide, polyacrylic acid, partial and complete alkali metal salts of polyacrylic acid, and the like. Also included are starch modified polyacrylic acids and hydrolyzed polyacrylonitrile and their alkali metal salts.

Useful superabsorbent polymers can be made by polymerizing acrylic acid and starch in an aqueous medium using a polyfunctional monomer, e.g., N,N-alkylene-bis-acrylamide, as the crosslinking agent. This process is described, for example, in U.S. Patent No. 4,076,663. Superabsorbent polymers can also be made as described in U.S. Patent No. 4,340,706 by the inverse polymerization of acrylic acid followed by crosslinking with a polyfunctional component, e.g., epichlorohydrin. Other superabsorbent polymers and processes for their manufacture are disclosed in U.S. Patent Nos. 4,654,039, 3,669,103 and 3,670,731.

The superabsorbent polymers particularly useful in this invention are those described in U.S. Patent Nos. 4,076,663 and 4,340,706. These superabsorbent polymers in particulate form have a particle size of 0.5 »m to 450 »m and are capable of absorbing at least 12 times their weight of an aqueous fluid. In a preferred embodiment superior absorption capabilities exist where the superabsorbent polymer in particulate form is less than 30 »m in size. These superabsorbent particulates swell when they absorb aqueous fluids. The particles maintain the approximate shape and geometry they had before contact with the fluid but the dimensions thereof are greatly enlarged.

In preparing the articles of this invention, other particulate materials which are insoluble in water and organic liquids but which are capable of absorbing, or adsorbing, liquids may be mixed with the superabsorbent polymer particulates. The superabsorbent polymer particulates may be mixed with the other particulate matter in an amount from 5 to 50 weight percent, and preferably 10 to 20 weight percent, of the superabsorbent particulates.

One example of other particulate matter is naturally occurring cellulose materials, such as saw dust, crushed corncobs, cotton linters and wood pulp. Another type of particulate matter which may be useful in this invention is silica gel which can adsorb fluids. Other useful adsorbents include molecular sieve zeolites, activated alumina and calcium sulfate, also known by the trade name Drierite®.

Ion-exchange resins can also be used as other particulate matter in this invention. Particularly useful ion-exchange resins are the strong acid, cation exchange resins.

Other particulate materials for use in this invention are clay minerals, such as kaolin, montmorillonite, illite, vermiculite, glauconite and attapulgite. These clay minerals are mixtures of metal oxides, e.g., aluminum oxide, magnesium oxide, potassium oxide, and silicon oxide and generally exist in the amophous state.

The superabsorbent particulate material (with or without other particulate material) is blended with a liquid to form a slurry-like mixture. Various liquids such as water, methanol, ethanol and other low alkyl alcohols can be used to form the slurry along with various other common organic solvents. While lower alkyl alcohols tend to Speed up the drying of the nonwoven wet-lay material, in a preferred embodiment, the liquid chosen to form the slurry is water since the preferred superabsorbent polymer particulates are insoluble in water and are not soluble in most common organic solvents.

The concentration of the superabsorbent polymer particulate in the slurry is not critical as long as a well mixed slurry is produced. In a preferred embodiment, because of the high water absorbent capacity of the superabsorbent particulate, the concentration of the superabsorbent polymer particulate in the water will be from 0.001 percent to 40 percent by weight and more preferably 0.01 percent to about 10 percent.

The superabsorbent polymer particulates are blended with the liquid by any conventional blending procedure including merely combining them in a container and stirring them by conventional stirring means to create a slurry-like blend. The particulates in the liquid should be stirred for 1 to 30 minutes to achieve better dispersion of the superabsorbent material.

Into this superabsorbent polymer particulate slurry are added fibers to form the wet-lay nonwoven material. Almost any conventional fibers, both synthetic and natural, can be used in this process, including polyester, polyethylene, polypropylene, polyvinyl alcohol, acrylic, acrylonitrile, nylon, polyurethane, rayon, tetrafluoroethylene, ethylene/tetrafluoroethylene, styrene-butadience rubber, rubber, triacetates, polyamides, polyvinylidens chlorides, polyvinyl chloride, polybenzimidazole, cellulose and wood pulp fibers. However, in a preferred embodiment, wood pulp fibers and common synthetic fibers are preferred since many of the natural fibers do not have a regular fiber length and, in addition, possess poor dispersion characteristics in the liquid. In a more preferred embodiment, the fibers for the mixture are chosen from the group including polyester, cellulose acetate (preferably non-water soluble cellulose acetates), polyvinyl alcohol, polyethylene, polypropylene, acrylic, glass, ceramic, mineral wool, and nylon fibers. If desired the fibers chosen can be a combination of two or more of the preferred fibers with the final choice of the desired fibers depending on the desired end product. For example, combinations of polyester and wood pulp fibers have shown great utility.

The length of the fiber chosen will also depend on the type of end product chosen. For example, if a strong nonwoven wet-lay material is desired, longer fiber lengths are preferred, generally an inch or longer. However, if softer less durable materials are desired, shorter fibers ranging from 3.125 to 25.4 mm (1/8 to one inch) in length should be chosen.

The concentration of the fibers to be added to the superabsorbent polymer particulate slurry will also vary significantly depending upon the degree of fluid absorbance required in the final material. The preferred superabsorbent material absorbs up to 360 grams of water per gram of the material and 150 grams of a one percent saline solution per gram of the material. Although the greater the percentage of the superabsorbent polymer in the final product the greater the absorption capacity of the end product, there are certain limitations on the amount of the superabsorbent material that can be combined with the fibers. If the percentage of the superabsorbent material combined with the fibers is too great, the nonwoven material will fall apart. Thus, in a preferred embodiment, the percentage of the superabsorbent polymer material in the end product should range from 0.1 percent to 60 percent and preferably from 1 to 25 percent.

There may also be added to the slurry mixture conventional adhesives to better hold the fibers together and to provide better tear resistance to the nonwoven material. In a preferred embodiment conventional adhesives such as acrylic, styrene-butadiene rubber, polyvinyl alcohol or nitrite adhesives may be added to the slurry mixture to bind the products together.

The fibers can be mixed with the superabsorbent polymer slurry by any conventional mixing means including merely adding the fibers gradually to the container holding the superabsorbent polymer particulate slurry and stirring the mixture. The superabsorbent polymer particulate slurry/fiber mixture should be well stirred to disperse the fibers throughout the mixture. In a preferred embodiment the superabsorbent polymer particulates slurry/fiber mixture should be stirred for 5 to 45 minutes with the amount of stirring dependent on the percentage of superabsorbent material in the mixtures.

After the fibers are well mixed, the mixture can be formed into any conventional product. In a preferred embodiment the mixture is poured onto a conventional screening mesh, porous enough to allow a significant percentage of the liquid in the slurry to be drained off without removing a significant amount of the fibers. The thickness of the mesh will depend on the particular fibers chosen and the product to be produced but in a preferred embodiment the mesh should be no larger than 0.59 mm (30 mesh). The superabsorbent particulate/fiber mixture can then be smoothed and thinned to any desired thickness. In a preferred embodiment the thickness of the mixture should be from 0.254 to 5.08 mm (10 to 200 mils).

After the mixture is poured onto the screen mesh and smoothed, it is dried by removing substantially all of the remaining liquid in the mixture. The mixture can be aired dried or it can be heated in an oven at any temperature less than 225°C. It is important that the temperature of the oven not exceed 250°C since the preferred superabsorbent particulate will begin to degrade at temperatures above 250°C.

The nonwoven wet-lay superabsorbent material produced by the process of this invention can be used as is in any conventional material where high fluid absorption is important. For example, the nonwoven superabsorbent material can be broken apart and used in sections in sanitary napkins, diapers, incontinence devices, outer wear lining, show liners, sweat bands, surgical sheets, food package liners, shipping package liners, medical culture shipping container liners, soil covers to prevent the growth of grass but to moisten soil, wound healing covering sheets and in other well known products in the industry.

In addition, a backing can be added to the nonwoven material to produce a sponge-like product, or products that will retain their shape after the absorption of moisture. In addition, the nonwoven material can be combined with other layered materials to form composites. These composites can be used in the industry in any of the well known applications for superabsorbent materials, such as food package liners, shipping package liners, outer wear liners, medical culture containers and other such conventional uses.

The following examples are given as specific illustrations of the invention. All parts and percentages are by weight unless otherwise stated. It is understood however that the invention is not limited to the specific details set forth in the examples.

### Example 1

A superabsorbent slurry was prepared by mixing 0.5 grams of a superabsorbent polymer particulate in 250 grams of water. The superabsorbent particulate was a graft copolymer of a 91 percent acrylic acid and 9 percent oxidized starch crosslinked with 0.1 percent N,N'-methylene-bis-acrylamide made by the process described in U.S. Patent No. 4,076,633. The mixture was stirred for 2 minutes in a conventional blender.

Added to this slurry were 10 grams of polyester fibers. The polyester fibers were from 12.7 to 38.1 mm (1/2 to 1-1/2 inches) in fiber length. The fiber/superabsorbent polymer particulate mixture was stirred for 2 minutes, in the blender. The mixture was then poured onto a 0.149 mm (100) mesh screen and spread out to a thickness of 12.7 to 19.05 mm (1/2 inch to 3/4 inch). The mixture was allowed to air dry at room temperature for 48 hours. The nonwoven wet-lay superabsorbent material produced by this process showed water absorbence of 55 grams per gram of the superabsorbent polymer material and 19 grams per gram of the superabsorbant polymer material in a 1 percent saline solution.

### Example 2

A superabsorbent slurry was prepared by mixing 1 gram of a superabsorbent polymer particulate in 250 ml of methyl alcohol in a conventional blender. The superabsorbent particulate was a graft copolymer of a 91 percent acrylic acid and 9 percent oxidized starch crosslinked with 0.1 percent N,N'-methylene-bis-acrylamide made by the process described in U.S. Patent No. 4,076,633. the mixture was stirred for 1 minute.

Added to this slurry were 9 grams of polyester fibers. The polyester fibers were less than 12.7 mm (1/2 inch) in fiber length. The polyester fiber/superabsorbent polymer particulate mixture was stirred for one minute in the blender, and then poured onto a 0.074 mm (200) screen mesh and spread out to a thickness of 12.7 to 19.05 mm (1/2 inch to 3/4 inch). The mixture was dried in a vacuum oven at a temperature of 100°C for 350 minutes. The nonwoven wet-lay polyester superabsorbent material produced by this process showed water absorbence of 59 grams per gram of the superabsorbent polymer material and 20 grams per gram of the superabsorbent polymer material in a 1 percent saline solution.

As is apparent from these Examples, high fluid absorbent nonwoven wet-lay materials can be produced by the process of this invention. These nonwoven materials can be used in sheets or can be broken apart into pieces and combined in a conventional superabsorbent applications such as diapers, sanitary napkins, incontinence materials, wound dressings, and other conventional fluid absorbent products. In addition, this material can be combined with other absorbent or wicking materials to form highly absorbent composite materials.

## Claims

1. A superabsorbent nonwoven wet-lay material obtainable by the process of:
a) blending superabsorbent polymer particulates with a liquid to form a superabsorbent polymer particulate slurry wherein the concentration of the superabsorbent material is from 0.001 to 40.0 wt% of the superabsorbent/liquid mixture, and wherein said superabsorbent polymer is a polymer of water soluble acrylic or vinyl monomers which are slightly crosslinked with a polyfunctional reactant;
b) mixing fibers with the superabsorbent polymer particulate slurry to form a superabsorbent polymer slurry/fiber mixture;
c) filtering the superabsorbent polymer slurry/fiber mixture to remove a portion of the liquid; and
d) drying the superabsorbent polymer fiber material at a temperature not in excess of 250°C to form a nonwoven wet-lay superabsorbent material.

2. The material of Claim 1 wherein the superabsorbent particulate material is a graft copolymer of acrylic acid and starch crosslinked with a disaturated monomer.

3. The material of Claim 1 wherein the fiber is selected from the group consisting of polyester, polyethylene, polypropylene, polyvinyl alcohol, acrylic, acrylonitrile, nylon, polyarylate, polyurethane, rayon, tetrafluoroethylene, ethylene/tetrafluoroethylene, styrene-butadiene rubber, triacetate, polyamides, polyvinyl chlorides, polyvinylidene chloride, polybenzimidazole fibers, wood pulp and cellulose fibers.

4. The material of Claim 3 wherein the fiber used is selected from the group comprising wood pulp, polyester, polypropylene, acrylic and nylon fibers.

5. The material of claim 1 wherein the fiber used is a combination of polyester and wood pulp fibers.

6. The material of claim 1 wherein more than one type of fiber is used with the superabsorbent polymer.

7. The material of Claim 1 wherein the concentration of the superabsorbent material is from 0.01 to 10.0 wt% of the superabsorbent/liquid mixture.

8. The material of Claim 1 wherein the liquid is water.

9. The material of Claim 1 wherein the liquid is a low-alkyl alcohol.

10. The material of Claim 1 wherein the superabsorbent particulate slurry/fiber mixture is dried at a temperature below 225°C.

11. The material of Claim 1 wherein the superabsorbent polymer material absorbs up to 350 grams per gram of water and up to 150 grams per gram of a 1 percent saline solution.

12. The material of Claim 1 wherein the percentage of the superabsorbent polymer material in the nonwoven wet-lay superabsorbant material is from 0.1 to 60 wt%.

13. The material of Claim 1 wherein the thickness of the nonwoven wet-lay superabsorbent polymer material is from 0.254 to 5.08 mm (10 to 200 mils).

14. The material of Claim 1 wherein the superabsorbent slurry/fiber mixture is mixed with other types of absorbent material.

15. The material of Claim 1 wherein the size of the superabsorbent polymer particulates are less than 30 »m.

16. A superabsorbent nonwoven wet-lay material prepared by the process according to claim 1, wherein the liquid in step a) is water, the fiber is selected from wood pulp, polyester, polypropylene, acrylic and nylon,
and drying of the superabsorbent polymer/fiber mixture is performed at a temperature less than 220°C.

17. A superabsorbent nonwoven wet-lay material prepared by the process according to claim 1, wherein the liquid in step a) is methyl alcohol, the fiber is selected from wood pulp and polyesters, and drying of the superabsorbent polymer/fiber mixture is performed at a temperature less than 220°C.

18. The material of Claim 16 wherein the percentage of the superabsorbent polymer material in the nonwoven wet-lay superabsorbant material is from 0.1 to 60 wt%.

19. The material of Claim 17 wherein the percentage of the superabsorbent polymer material in the nonwoven wet-lay superabsorbant material is from 0.1 to 60 wt%.

20. The material of Claim 16 wherein the superabsorbent particulate material is a graft copolymer of acrylic acid and starch crosslinked with a disaturated monomer.

21. The material of Claim 17 wherein the superabsorbent particulate material is a graft copolymer of acrylic acid and starch crosslinked with a disaturated monomer.

22. The material of Claim 16 wherein the concentration of the superabsorbent material is from 0.01 to 10.0 wt% of the superabsorbent/liquid mixture.

23. The material of Claim 17 wherein the concentration of the superabsorbent material is from 0.01 to 10.0 wt% of the superabsorbent/liquid mixture.

24. The material of Claim 16 wherein the superabsorbent polymer material absorbs up to 350 grams per gram of water and up to 150 grams per gram of a 1 percent saline solution.

25. The material of Claim 17 wherein the superabsorbent polymer material absorbs up to 350 grams per gram of water and up to 150 grams per gram of a 1 percent saline solution.

26. The material of Claim 16 wherein the size of the superabsorbent polymer particulates are less than 30 »m.

27. The material of Claim 17 wherein the size of the superabsorbent polymer particulates are less than 30 »m.

## Patentansprüche

1. Hochabsorbierendes Naßlege-Vliesmaterial, erhältlich durch das Verfahren
a) des Mischens eines hochabsorbierenden Polymers in Teilchenform mit einer Flüssigkeit zur Bildung einer Aufschlämmung der hochabsorbierenden Polymer-Teilchen, worin die Konzentration des hochabsorbierenden Materials 0,001 bis 40 Gew.-% des Gemischs aus hochabsorbierendem Material und Flüssigkeit beträgt und worin das hochabsorbierende Polymer ein Polymer aus wasserlöslichen Acryl- oder Vinyl-Monomeren ist, die mit einem mehrfunktionellen Reaktionspartner schwach vernetzt sind;
b) des Vermischens von Fasern mit der Aufschlämmung der hochabsorbierenden Polymer-Teilchen zur Bildung eines Gemischs aus der Aufschlämmung des hochabsorbierenden Polymers und den Fasern;
c) des Filtrierens des Gemischs aus der Aufschlämmung des hochabsorbierenden Polymers und den Fasern, um einen Teil der Flüssigkeit zu entfernen; und
d) des Trocknens des hochabsorbierenden Polymer-Faser-Materials bei einer Temperatur nicht über 250 °C zur Bildung eines hochabsorbierenden Naßlege-Vliesmaterials.

2. Material nach Anspruch 1, worin das hochabsorbierende teilchenförmige Material ein Pfropfpolymer aus Acrylsäure und Stärke ist, das mit einem doppelt abgesättigten Monomer vernetzt ist.

3. Material nach Anspruch 1, worin die Faser aus der aus Polyester-, Polyethylen-, Polypropylen-, Polyvinylalkohol-, Acryl-, Acrylnitril-, Nylon-, Polyarylat-, Polyurethan-, Reyon-, Tetrafluorethylen-, Ethylen/Tetrafluorethylen-, Styrol-Butadien-Kautschuk-, Triacetat-, Polyamid-, Polyvinylchlorid-, Polyvinylidenchlorid-, Polybenzimidazol-Fasern, Holzzellstoff- und Cellulose-Fasern bestehenden Gruppe ausgewählt ist.

4. Material nach Anspruch 3, worin die eingesetzte Faser aus der aus Holzzellstoff-, Polyester-, Polypropylen-, Acryl- und Nylon-Fasern bestehenden Gruppe ausgewählt ist.

5. Material nach Anspruch 1, worin die eingesetzte Faser eine Kombination aus Polyester- und Holzzellstoff-Fasern ist.

6. Material nach Anspruch 1, worin mehr als ein Faser-Typ mit dem hochabsorbierenden Polymer verwendet wird.

7. Material nach Anspruch 1, worin die Konzentration des hochabsorbierenden Materials 0,01 bis 10,0 Gew.-% des Gemischs aus hochabsorbierendem Material und Flüssigkeit beträgt.

8. Material nach Anspruch 1, worin die Flüssigkeit Wasser ist.

9. Material nach Anspruch 1, worin die Flüssigkeit ein Niederalkylalkohol ist.

10. Material nach Anspruch 1, worin das Gemisch aus der Aufschlämmung des hochabsorbierenden Polymers und den Fasern bei einer Temperatur unter 225 °C getrocknet wird.

11. Material nach Anspruch 1, worin 1 g des hochabsorbierenden Polymer-Materials bis zu 350 g Wasser und bis zu 150 g einer 1-proz. Kochsalz-Losung absorbiert.

12. Material nach Anspruch 1, worin der prozentuale Anteil des hochabsorbierenden Polymer-Materials in dem hochabsorbierenden Naßlege-Vliesmaterial 0,1 bis 60 Gew.-% beträgt.

13. Material nach Anspruch 1, worin die Dicke des hochabsorbierenden Naßlege-Vliesmaterial 0,254 bis 5,08 mm (10 bis 200 mil) beträgt.

14. Material nach Anspruch 1, worin das Gemisch aus der Aufschlämmung des hochabsorbierenden Polymers und den Fasern mit anderen Typen eines absorbierenden Materials vermischt wird.

15. Material nach Anspruch 1, worin die Größe der Teilchen des hochabsorbierenden Polymers kleiner als 30 »m ist.

16. Hochabsorbierendes Naßlege-Vliesmaterial, hergestellt mittels des Verfahrens gemäß Anspruch 1, worin die Flüssigkeit in Schritt a) Wasser ist, die Faser aus Holzzellstoff, Polyester, Polypropylen, Acryl und Nylon ausgewählt ist und das Trocknen des Gemischs aus dem hochabsorbierenden Polymer und den Fasern bei einer Temperatur von weniger als 220 °C durchgeführt wird.

17. Hochabsorbierendes Naßlege-Vliesmaterial, hergestellt mittels des Verfahrens gemäß Anspruch 1, worin die Flüssigkeit in Schritt a) Methylalkohol ist, die Faser aus Holzzellstoff und Polyester ausgewählt ist und das Trocknen des Gemischs aus dem hochabsorbierenden Polymer und den Fasern bei einer Temperatur von weniger als 220 °C durchgeführt wird.

18. Material nach Anspruch 16, worin der prozentuale Anteil des hochabsorbierenden Polymer-Materials in dem hochabsorbierenden Naßlege-Vliesmaterial 0,1 bis 60 Gew.-% beträgt.

19. Material nach Anspruch 17, worin der prozentuale Anteil des hochabsorbierenden Polymer-Materials in dem hochabsorbierenden Naßlege-Vliesmaterial 0,1 bis 60 Gew.-% beträgt.

20. Material nach Anspruch 16, worin das hochabsorbierende teilchenförmige Material ein Pfropfpolymer aus Acrylsäure und Stärke ist, das mit einem doppelt abgesättigten Monomer vernetzt ist.

21. Material nach Anspruch 17, worin das hochabsorbierende teilchenförmige Material ein Pfropfpolymer aus Acrylsäure und Stärke ist, das mit einem doppelt abgesättigten Monomer vernetzt ist.

22. Material nach Anspruch 16, worin die Konzentration des hochabsorbierenden Materials 0,01 bis 10,0 Gew.-% des Gemischs aus hochabsorbierendem Material und Flüssigkeit beträgt.

23. Material nach Anspruch 17, worin die Konzentration des hochabsorbierenden Materials 0,01 bis 10,0 Gew.-% des Gemischs aus hochabsorbierendem Material und Flüssigkeit beträgt.

24. Material nach Anspruch 16, worin 1 g des hochabsorbierenden Polymer-Material bis zu 350 g Wasser und bis zu 150 g einer 1-proz. Kochsalz-Lösung absorbiert.

25. Material nach Anspruch 17, worin 1 g des hochabsorbierenden Polymer-Material bis zu 350 g Wasser und bis zu 150 g einer 1-proz. Kochsalz-Lösung absorbiert.

26. Material nach Anspruch 16, worin die Größe der Teilchen des hochabsorbierenden Polymers kleiner als 30 »m ist.

27. Material nach Anspruch 17, worin die Größe der Teilchen des hochabsorbierenden Polymers kleiner als 30 »m ist.

## Revendications

1. Matière non tissée hyperabsorbante pour couchage par voie humide, pouvant être obtenue par le procédé consistant :
a) à mélanger des particules polymériques hyperabsorbantes à un liquide pour former une suspension de particules polymériques hyperabsorbantes dont la concentration en matière hyperabsorbante est comprise dans l'intervalle de 0,001 à 40,0 % en poids du mélange matière hyperabsorbante/liquide, et dans laquelle ledit polymère hyperabsorbant est un polymère de monomères acryliques ou vinyliques hydrosolubles qui sont légèrement réticulés avec un corps réactionnel polyfonctionnel ;
b) à mélanger des fibres à la suspension de particules polymériques hyperabsorbantes pour former un mélange suspension de polymère hyperabsorbant/fibres ;
c) à filtrer le mélange suspension de polymère hyperabsorbant/fibres pour éliminer une portion du liquide ; et
d) à sécher la matière constituée du polymère hyperabsorbant et des fibres à une température non supérieure à 250°C pour former une matière hyperabsorbante non tissée pour couchage par voie humide.

2. Matière suivant la revendication 1, dans laquelle les particules hyperabsorbantes sont constituées d'un polymère greffé d'acide acrylique et d'amidon réticulé avec un monomère doublement saturé.

3. Matière suivant la revendication 1, dans laquelle les fibres sont choisies dans le groupe consistant en fibres de polyester, de polyéthylène, de polypropylène, de polymère d'alcool vinylique, de résine acrylique, d'acrylonitrile, de Nylon, de polyarylate, de polyuréthanne, de rayonne, de tétrafluoréthylène, de polymère éthylène/tétrafluoréthylène, de caoutchouc styrène-butadiène, de triacétate, de polyamides, de polymères de chlorure de vinyle, de polymère de chlorure de vinylidène, de polybenzimidazole, fibres de pâte de bois et de fibres de cellulose.

4. Matière suivant la revendication 3, dans laquelle les fibres utilisées sont choisies dans le groupe consistant en des fibres de pâte de bois, de polyester, de polypropylène, de résine acrylique et de Nylon.

5. Matière suivant la revendication 1, dans laquelle les fibres utilisées consistent en une association de fibres de polyester et de fibres de pâte de bois.

6. Matière suivant la revendication 1, dans laquelle plus d'un type de fibres est utilisé avec le polymère hyperabsorbant.

7. Matière suivant la revendication 1, dans laquelle la concentration de la matière hyperabsorbante est comprise dans l'intervalle de 0,01 à 10,0 % en poids du mélange matière hyperabsorbante/liquide.

8. Matière suivant la revendication 1, dans laquelle le liquide est l'eau.

9. Matière suivant la revendication 1, dans laquelle le liquide est un alcool alkylique inférieur.

10. Matière suivant la revendication 1, dans laquelle le mélange suspension de particules hyperabsorbantes/fibres est séché à une température inférieure à 225°C.

11. Matière suivant la revendication 1, dans laquelle le polymère hyperabsorbant absorbe jusqu'à 350 grammes d'eau par gramme et jusqu'à 150 grammes d'une solution à 1 % de sel par gramme.

12. Matière suivant la revendication 1, dans laquelle le pourcentage du polymère hyperabsorbant dans la matière hyperabsorbante non tissée pour couchage par voie humide est compris dans l'intervalle de 0,1 à 60 % en poids.

13. Matière suivant la revendication 1, dans laquelle l'épaisseur de la matière polymérique hyperabsorbante non tissée pour couchage par voie humide est comprise dans l'intervalle de 0,254 à 5,08 mm (10 à 200 mils).

14. Matière suivant la revendication 1, dans laquelle le mélange suspension de polymère hyperabsorbant/fibres est mélangé à d'autres types de matière absorbante.

15. Matière suivant la revendication 1, dans laquelle les dimensions des particules de polymère hyperabsorbant sont inférieures à 30 »m.

16. Matière non tissée hyperabsorbante pour couchage par voie humide préparée par le procédé suivant la revendication 1, dans laquelle le liquide dans l'étape a) est l'eau, les fibres sont choisies entre des fibres de pâte de bois, de polyester, de polypropylène, de résine acrylique, de Nylon, le séchage du mélange polymère hyperabsorbant/fibres étant effectué à une température inférieure à 220°C.

17. Matière non tissée hyperabsorbante pour couchage par voie humide préparée par le procédé suivant la revendication 1, dans laquelle le liquide dans l'étape a) est l'alcool méthylique, les fibres sont choisies entre des fibres de pâte de bois et des fibres de polyester, et le séchage du mélange polymère hyperabsorbant/fibres est effectué à une température inférieure à 220°C.

18. Matière suivant la revendication 16, dans laquelle le pourcentage du polymère hyperabsorbant dans la matière hyperabsorbante non tissée pour couchage par voie humide est compris dans l'intervalle de 0,1 à 60 % en poids.

19. Matière suivant la revendication 17, dans laquelle le pourcentage du polymère hyperabsorbant dans la matière hyperabsorbante non tissée pour couchage par voie humide est compris dans l'intervalle de 0,1 à 60 % en poids.

20. Matière suivant la revendication 16, dans laquelle la matière hyperabsorbante en particules est un copolymère greffé d'acide acrylique et d'amidon réticulé avec un monomère doublement saturé.

21. Matière suivant la revendication 17, dans laquelle la matière hyperabsorbante en particules est un copolymère greffé d'acide acrylique et d'amidon réticulé avec un monomère doublement saturé.

22. Matière suivant la revendication 16, dans laquelle la concentration de la matière hyperabsorbante est comprise dans l'intervalle de 0,01 à 10,0 % en poids du mélange matière hyperabsorbante/liquide.

23. Matière suivant la revendication 17, dans laquelle la concentration de la matière hyperabsorbante est comprise dans l'intervalle de 0,01 à 10,0 % en poids du mélange matière hyperabsorbante/liquide.

24. Matière suivant la revendication 16, dans laquelle le polymère hyperabsorbant absorbe jusqu'à 350 grammes d'eau par gramme et jusqu'à 150 grammes d'une solution à 1 % de sel par gramme.

25. Matière suivant la revendication 17, dans laquelle le polymère hyperabsorbant absorbe jusqu'à 350 grammes d'eau par gramme et jusqu'à 150 grammes d'une solution à 1 % de sel par gramme.

26. Matière suivant la revendication 16, dans laquelle les dimensions des particules de polymère hyperabsorbant sont inférieures à 30 »m.

27. Matière suivant la revendication 17, dans laquelle les dimensions des particules du polymère hyperabsorbant sont inférieures à 30 »m.
